# EUROPEAN PATENT SPECIFICATION

(11) **EP 3 318 279 B1**
(45) Date of publication and mention of the grant of the patent: **25.02.2026**
(21) Application number: 17199531.9
(22) Date of filing: 01.11.2017
(51) Int. Cl.: A61K 47/10, A61K 47/26, A61K 47/38, A61K 9/20, A61K 31/496

(54) **SOLID ORAL PHARMACEUTICAL COMPOSITIONS OF LURASIDONE HYDROCHLORIDE**
FESTE ORALE PHARMAZEUTISCHE ZUSAMMENSETZUNGEN VON LURASIDON-HYDROCHLORID
COMPOSITIONS PHARMACEUTIQUES ORALES SOLIDES DE CHLORHYDRATE DE LURASIDONE

(30) Priority: 02.11.2016 TR 201615609
(43) Date of publication of application: 09.05.2018
(73) Proprietor: Sanovel Ilac Sanayi ve Ticaret A.S., Sisli - Istanbul (TR)
(72) Inventor: TÜRKYILMAZ, Ali, 34460 Istanbul (TR); PEHLIVAN AKALIN, Nur, 34460 Istanbul (TR); ERGUN DONMEZ, Merve, 34460 Istanbul (TR)
(74) Representative: Genç Ilhan, Oznur

(56) References cited:
- EP-A1- 1 884 242
- WO-A1-2012/156981
- WO-A1-2016/012898
- WO-A2-2014/076712
- US-A1- 2014 343 076
- US-A1- 2015 157 628

## Description

### Field of Invention

The present invention relates to pharmaceutical compositions, which provide a better dissolution and disintegration profile, comprising lurasidone hydrochloride with at least one binder and one or more other pharmaceutically acceptable excipient.

### Background of Invention

Atypical antipsychotics are newer antipsychotic agents that have a pharmacological profile different from older or typical antipsychotic drugs. They are also called second generation antipsychotics. The drugs in this class of antipsychotics act on many receptor types including dopamine and serotonin, but they are more selective for dopamine receptors. They cause less extrapyramidal side effects compared to the older typical antipsychotic drugs. They are more effective in treatment-resistant patients and have a greater efficacy to treat negative symptoms, compared to the typical antipsychotics.

Lurasidone is an atypical antipsychotic which belongs to the chemical class of benzisothiazol derivatives. Its chemical name is (3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione and its chemical structure is shown in the Formula 1.

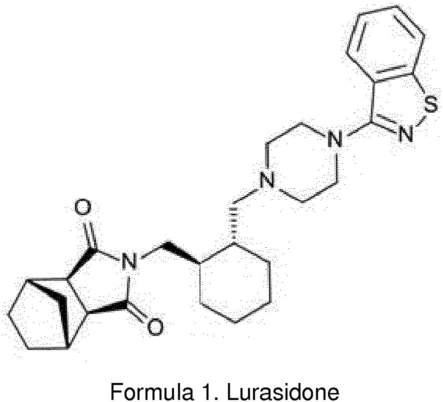

Lurasidone hydrochloride is a salt of lurasidone which is developed by Dainippon Sumitomo under the trade name Latuda^{®} which is indicated for the treatment of patients with schizophrenia and with major depressive episodes associated with bipolar I disorder (bipolar depression).

Lurasidone hydrochloride is a white to off-white powder. It is very slightly soluble in water, practically insoluble or insoluble in 0.1 N HCl, slightly soluble in ethanol, sparingly soluble in methanol, practically insoluble or insoluble in toluene and very slightly soluble in acetone.

In the state of the art, it is disclosed a pharmaceutical composition of lurasidone which is free of binder and which can also exhibit rapid disintegration as well as equivalent in vitro dissolution profile. On the other hand, it is stated that the pharmaceutical composition comprises pregelatinized starch in the ratio of less than 10% by weight. In the light of the literature information given in the Handbook of Pharmaceutical Excipients edited by Raymond C. Rowe, Paul J. Sheskey and Marlan E. Quinn (sixth edition, page 692), it is known that pregelatinized starch can be used as a binder and/or as a disintegrant in a tablet formulation between the ratios of 5-10% by weight. Therefore, it is understood that the pregelatinized starch is present as disintegrant in this pharmaceutical composition.

In the patent application no. WO2016012898A1, an oral pharmaceutical composition of lurasidone, comprising a single disintegrant which is free of starch and its derivatives, is mentioned. It is also stated that this composition exhibits an equivalent dissolution even when the content of lurasidone is varied. Furthermore, lactose monohydrate is used in the composition as binder instead of pregelatinized starch and its ratio in the total formulation is about 24% which can be inconvenient for lactose-intolerant patients in long term use. On the other hand, pregelatinized starch is generally regarded as a nontoxic and nonirritant excipient upon the information given in the Handbook of Pharmaceutical Excipients (sixth edition, page 693) and use of pregelatinized starch in a tablet formulation is known to remarkably improve the disintegration and the dissolution profiles of a pharmaceutical composition. So the said application is not specific about the advantages provided via the absence of starch derivatives.

EP1884242A1 relates to lurasidone-containing compositions that further comprise pregelatinized starch, a water-soluble excipient, and a water-soluble polymer binder. The primary objective of EP1884242A1 is to achieve an equivalent dissolution profile of lurasidone regardless of the amount of active ingredient. EP1884242A1 highlights that obtaining an equivalent dissolution profile becomes more challenging when the active ingredient is present in high concentrations. The proposed solution, as described in paragraph [0012] of EP1884242A1, involves the use of pregelatinized starch, a water-soluble excipient, and a water-soluble polymer binder. Paragraph [0012] further specifies that the water-soluble excipient may be mannitol or lactose. According to paragraph [0018], examples of suitable water-soluble polymer binders include hydroxypropylcellulose, hydroxypropyl methylcellulose, polyvinylpyrrolidone, polyvinyl alcohol, among others.

Yet another patent application no. US2015157628A1 asserts pharmaceutical compositions comprising lurasidone and starch derivatives in lower ratios in comparison with the state of art and aims to decrease the weight of a tablet. However, the recommended amounts of lurasidone and starch derivatives are rather high, between 20-50% and more than 50% by weight of the composition, respectively. Moreover, in the preparation process of this composition, only water is used to form binder/granulation solution which gives rise to undesired long dry time.

Lurasidone has a low aqueous solubility (0.00789 mg/ml in water) and when used in micronized form, especially in solid oral pharmaceutical compositions, it is challenging to process due to its sticky nature. Thus, there is a need in the art for a pharmaceutical composition comprising lurasidone providing a better dissolution and disintegration profile by means of the well-proportioned use of a well-selected disintegrant and binders which reduces the required content of lurasidone and starch derivatives accordingly.

### Objects and Brief Description of the Invention

The main object of the present invention is to obtain lurasidone hydrochloride formulations eliminating all aforesaid problems and bringing additional advantages to the relevant prior art.

Another object of the present invention is to obtain lurasidone hydrochloride formulations with high stability and bioavalibility.

A further object of the present invention is to develop solid oral formulations of lurasidone hydrochloride having an improved level of dissolution rate and solubility.

According to these objects, another object of the present invention is to obtain a solid oral pharmaceutical composition comprising lurasidone hydrochloride and at least one binder.

Yet, another object of the present invention is to provide a lurasidone hydrochloride tablet comprising at least one film coating to protect the pharmaceutical composition against the moisture to maintain the stability.

A further object of the present invention is to improve a process for preparing the said lurasidone hydrochloride tablet, comprising spray granulation method which makes round and compact granules having a high bulk density and a narrower range of particle sizes.

### Detailed Description of the Invention

In accordance with the objects outlined above, detailed features of the present invention are given herein.

A solid oral pharmaceutical composition subjected to the invention essentially comprises lurasidone or its pharmaceutically acceptable salts as an active agent and at least one binder.

According to the invention the active agent is lurasidone hydrochloride.

The amount of lurasidone hydrochloride is between 5-30% by weight of the total composition. More preferably the amount of lurasidone hydrochloride is between 10-20% by weight of the total composition.

According to this embodiment, lurasidone hydrochloride is present in an amount of between 1 to 300 mg, preferably 10 to 240 mg and more preferably it is 20 to 120 mg.

According to these embodiments, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

The composition is preferably in the form of a film-coated tablet.

According to the invention, the solid oral pharmaceutical composition comprises at least two binders which are pregelatinized starch and polyvinylpyrrolidone.

The ratio of pregelatinized starch to the total weight of lurasidone hydrochloride is in the range of 1:2 to 1:3.

The amount of pregelatinized starch is between 1-15%, preferably 5-10% by weight of the total composition. Due to its amount in the composition, pregelatinized starch also takes part in disintegration of the film-coated tablet even it is used as a binder.

The ratio of pregelatinized starch to lurasidone and the ratio of pregelatinized starch in the total composition are reduced and even so a better dissolution and disintegration profile and a better stability are obtained over the prior art.

The amount of polyvinylpyrrolidone is between 1-10%, preferably 3-7% by weight of the total composition.

According to one embodiment, the composition further comprises at least one pharmaceutically acceptable excipient selected from diluents, disintegrants, lubricants, or mixtures thereof.

According to this embodiment, the solid oral pharmaceutical composition comprises at least one diluent which is selected from the group comprising lactose monohydrate, lactose, dibasic calcium phosphate, microcrystalline cellulose, mannitol, spray-dried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment, the solid oral pharmaceutical composition comprises two diluents which are mannitol and microcrystalline cellulose.

The amount of mannitol is between 5-60%, preferably 15-40% and more preferably 25-35% by weight of the total composition.

The amount of microcrystalline cellulose is between 5-60%, preferably 15-40% and more preferably 20-30% by weight of the total composition. According to this preferred ratio selection, it is possible to benefit the binding effect of the microcrystalline cellulose which is used as a diluent.

According to the invention, the solid oral pharmaceutical composition comprises at least one disintegrant which is is croscarmellose sodium. The amount of croscarmellose sodium is between 1:2 to 1:3.

According to this embodiment, the ratio of croscarmellose sodium to the total weight of binder is in the range of 1:0.5 to 1:10 and preferably 1:1 to 1:3 and more preferably 1:1 to 1:2.

The ratios of croscarmellose sodium to the other excipients are very important for this invention to improve the quality of the film-coated tablet, since it is known that disintegrants have tablet quality deteriorating properties, such as causing tablet hardness reductions and tablet surface roughness as a result of moisture absorption, and worsening the mouth touch due to a feeling of dryness as a result of saliva absorption. Regarding this knowledge, in this invention, corscarmellose sodium is used in minimal ratios as given above as the single disintegrant.

According to these embodiments, the solid oral pharmaceutical composition comprises a lubricant which is selected from the group comprising sodium lauryl sulphate, sodium stearyl fumarate, magnesium stearate, zinc stearate, calcium stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin or mixtures thereof.

According to the preferred embodiment, lubricant used in this present invention is magnesium stearate.

The amount of magnesium stearate is between 0.1-5 %, preferably 0.1-2% by weight of the total composition.

According to these embodiments, the solid oral pharmaceutical composition comprises at least one coating layer to protect the composition against the moisture and maintain the stability. Suitable coating ingredients are selected from the group comprising hydroxypropylmethyl cellulose (hypromellose), hydroxypropyl cellulose, polyvinyl alcohol (PVA), polyethylene glycol (PEG), talc, polyvinyl alcohol-polyethylene glycol copolymers (Kollicoat IR), ethylcellulose dispersions (Surelease), polyvinylprolidone, polyvinylprolidone-vinyl acetate copolymer (PVP-VA) and all kinds of OpadryTM, pigments, dyes, titanium dioxide, iron oxide or polymethylmetacrylate copolymers (Eudragit) and mixtures thereof.

According to one embodiment, coating layer is Opadry White which comprises hypromellose, titanium dioxide, talc and polyethylene glycol.

According to another embodiment; coating layer is Opadry Green which comprises hypromellose, titanium dioxide, talc, polyethylene glycol, iron oxide (yellow dye), indigo carmine aluminum lake (blue dye).

According to one preferred embodiment, the solid oral pharmaceutical composition comprises lurasidone hydrochloride as active agent, pregelatinized starch and polyvinylpyrrolidone as binders, microcrystalline cellulose and mannitol as diluents, croscarmellose sodium as disintegrant, magnesium stearate as lubricant and a coating layer.

According to this preferred embodiment,
- the amount of lurasidone hydrochloride is between 5-30%
- the amount of pregelatinized starch is between 1-15%,
- the amount of polyvinylpyrrolidone is between 1-10%,
- the amount of microcrystalline cellulose is between 5-60%,
- the amount of croscarmellose sodium is between 1-15%,
- the amount of mannitol is between 5-60%,
- the amount of magnesium stearate is between 0.1-5%,
- the amount of the coating layer is between 1-5%, by weight of the total composition.

These analytically selected ratios ensure the required effective doses for the treatment and enhance the stability and dissolution profile of the film-coated tablet subjected to the invention.

According to all these embodiments, the below given formulations can be used in the solid oral pharmaceutical composition subjected to the invention.

### Example 1: Opadry white coated tablet

| **Ingredients** | **Amount (%)** |
|---|---|
| Lurasidone hydrochloride | 10 - 20 |
| Pregelatinized starch | 5 - 10 |
| Polyvinylpyrrolidone (K25) | 3 - 7 |
| Microcrystalline cellulose (Avicel 101) | 20 - 30 |
| Croscarmellose sodium | 5 - 10 |
| Mannitol (Pearlitol 200SD) | 25 - 35 |
| Magnesium stearate | 0.1 - 2 |
| Coating | 1 - 5 |

| **Coating Material (Opadry White) Ingredients** | **Amount (%)** |
|---|---|
| Hypromellose (Methocel E5 LV) | 60 - 80 |
| Titanium dioxide | 15 - 25 |
| Talc | 3 - 10 |
| Polyethylene glycol powder (PEG 8000) | 2 - 5 |

### Example 2: Opadry green coated tablet

| **Ingredients** | **Amount (%)** |
|---|---|
| Lurasidone hydrochloride | 10-20 |
| Pregelatinized starch | 5 - 10 |
| Polyvinylpyrrolidone (K25) | 3 - 7 |
| Microcrystalline cellulose (Avicel 101) | 20 - 30 |
| Croscarmellose sodium | 5 - 10 |
| Mannitol (Pearlitol 200SD) | 25 - 35 |
| Magnesium stearate | 0.1 - 2 |
| Coating | 1 - 5 |

| **Coating Material (Opadry Green) Ingredients** | **Amount (%)** |
|---|---|
| Hypromellose (Methocel E5 LV) | 60 - 80 |
| Titanium dioxide | 10 - 20 |
| Talc | 3 - 10 |
| Polyethylene glycol powder (PEG 8000) | 2 - 5 |
| Iron oxide (yellow dye) | 1 - 3 |
| Indigo carmine aluminum lake (blue dye). | 1 - 3 |

### Example 3: A preferred coated Tablet

| **Ingredients** | **Amount (%)** |
|---|---|
| Lurasidone hydrochloride | 18 |
| Pregelatinized starch | 8 |
| Polyvinylpyrrolidone (K25) | 5.3 |
| Microcrystalline cellulose (Avicel 101) | 24.8 |
| Croscarmellose sodium | 8 |
| Mannitol (Pearlitol 200SD) | 33 |
| Magnesium stearate | 0.5 |
| Coating | 2.4 |

The pharmaceutical formulations mentioned above are prepared by following these steps:
- swelling polyvinylpyrrolidone in a 70% alcohol solution comprising ethanol and water, thus preparing a granulation solution,
- mixing microcrystalline cellulose, pregelatinized starch, croscarmellose sodium and lurasidone hydrochloride in a fluid bed dryer for 10 minutes to prepare a powder mixture,
- granulating the powder mixture with the granulation solution using spray granulation method,
- drying the granules, preferably until they have 3% moisture ratio.
- sieving the granules preferably with a 0,7mm sieving mesh and grinding them,
- mixing the grinded granules with mannitol for 15 minutes in a container mixer,
- adding magnesium stearate to the mannitolized granules and mixing for 3 more minutes,
- weighing the final mixture and compress into tablets,
- coating these tablets with a film layer

Spray granulation method which is performed to granulate the powder mixture results in granules with a round, compact structure, excellent flow properties, a high bulk density and a narrower range of particle sizes. These properties make easier to process further. In addition to this, spray granulation with fluid bed technology enables to choose residual moisture and particle size. As a result of these advantages, the tablet composition has an enhanced stability during its shelf-life.

## Claims

1. A solid oral pharmaceutical composition comprising lurasidone hydrochloride, at least one disintegrant and two binders which are pregelatinized starch and polyvinylpyrrolidone; wherein the ratio of pregelatinized starch to the total weight of lurasidone hydrochloride is in the range of 1:2 to 1:3 and wherein said disintegrant is croscarmellose sodium and wherein the ratio of croscarmellose sodium to the total weight of lurasidone hydrochloride is in the range of 1:2 to 1:3.

2. The solid oral pharmaceutical composition according to claim 1, wherein the composition comprises lurasidone hydrochloride in an amount of 5-30% and more preferably 10-20% by weight.

3. The solid oral pharmaceutical composition according to claim 1 or 2, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

4. The solid oral pharmaceutical composition according to claim 3, wherein the composition is in the form of a film-coated tablet.

5. The solid oral pharmaceutical composition according to any preceding claims, wherein the composition further comprises at least one pharmaceutically acceptable excipient selected from diluents, disintegrants, lubricants, or mixtures thereof.

6. The solid oral pharmaceutical composition according to claim 1 wherein the ratio of croscarmellose sodium to the total weight of binder is in the range of 1:0.5 to 1:10 and preferably 1:1 to 1:3 and more preferably 1:1 to 1:2.

7. The solid oral pharmaceutical composition according to any preceding claims, wherein the composition comprises;
- 5-30% by weight lurasidone hydrochloride,
- 1-15% by weight of pregelatinized starch,
- 1-10% by weight of polyvinylpyrrolidone,
- 5-60% by weight of microcrystalline cellulose,
- 1-15% by weight of croscarmellose sodium,
- 5-60% by weight of mannitol,
- 0.1-1% by weight of magnesium stearate
- 1-5% by weight of moisture protective coating.

8. A process for preparing the solid oral pharmaceutical composition according to claim 7 comprising the following steps:
- swelling polyvinylpyrrolidone in a 70% alcohol solution comprising ethanol and water, thus preparing a granulation solution,
- mixing microcrystalline cellulose, pregelatinized starch, croscarmellose sodium and lurasidone hydrochloride in a fluid bed dryer for 10 minutes to prepare a powder mixture,
- granulating the powder mixture with the granulation solution using spray granulation method,
- drying the granules
- sieving the granules and grinding them,
- mixing the grinded granules with mannitol for 15 minutes in a container mixer,
- adding magnesium stearate to the mannitolized granules and mixing for 3 more minutes,
- weighing the final mixture and compress into tablets,
- coating these tablet with a film layer.

## Patentansprüche

1. Eine feste orale pharmazeutische Zusammensetzung, umfassend Lurasidonhydrochlorid, mindestens ein Sprengmittel und zwei Bindemittel, die vorgelatinierte Stärke und Polyvinylpyrrolidon sind; wobei das Verhältnis von vorgelatinierter Stärke zum Gesamtgewicht von Lurasidonhydrochlorid im Bereich von 1:2 bis 1:3 liegt und wobei das Sprengmittel Croscarmellose-Natrium ist und wobei das Verhältnis von Croscarmellose-Natrium zum Gesamtgewicht von Lurasidonhydrochlorid im Bereich von 1:2 bis 1:3 liegt.

2. Die feste orale pharmazeutische Zusammensetzung gemäß Anspruch 1, wobei die Zusammensetzung Lurasidonhydrochlorid in einer Menge von 5-30 Gew.-% und vorzugsweise 10-20 Gew.-% umfasst.

3. Die feste orale pharmazeutische Zusammensetzung gemäß Anspruch 1 oder 2, wobei die Zusammensetzung in Form einer beschichteten Tablette, einer dreischichtigen Tablette, einer zweischichtigen Tablette, einer mehrschichtigen Tablette, einer oral zerfallenden Tablette, Minitablette, Pellet, Zuckerpellet, Buccaltablette, Sublingualtablette, Brausetablette, Tablette mit sofortiger Freisetzung, Tablette mit modifizierter Freisetzung, Filmtablette, magensaftlösliche Tablette, Pille, Kapsel, orales Granulat, Pulver, beschichtetes Kügelchensystem, Mikrosphäre, Tablette in Tablette, Inlay-Tablette, Dragee, Sachet oder oral verabreichbarer Film.

4. Die feste orale pharmazeutische Zusammensetzung gemäß Anspruch 3, wobei die Zusammensetzung in Form einer Filmtablette vorliegt.

5. Die feste orale pharmazeutische Zusammensetzung gemäß einem der vorstehenden Ansprüche, wobei die Zusammensetzung ferner mindestens einen pharmazeutisch akzeptablen Hilfsstoff umfasst, der aus Verdünnungsmitteln, Sprengmitteln, Gleitmitteln oder Mischungen davon ausgewählt ist.

6. Die feste orale pharmazeutische Zusammensetzung gemäß Anspruch 1, wobei das Verhältnis von Croscarmellose-Natrium zum Gesamtgewicht des Bindemittels im Bereich von 1:0,5 bis 1:10 und vorzugsweise 1:1 bis 1:3 und noch bevorzugter 1:1 bis 1:2 liegt.

7. Die feste orale pharmazeutische Zusammensetzung gemäß einem der vorstehenden Ansprüche, wobei die Zusammensetzung umfasst:
- 5-30 Gew.-% Lurasidonhydrochlorid,
- 1-15 Gew.-% vorgelatinierte Stärke,
- 1-10 Gew.-% Polyvinylpyrrolidon,
- 5-60 Gew.-% mikrokristalline Cellulose,
- 1-15 Gew.-% Croscarmellose-Natrium,
- 5-60 Gew.-% Mannitol,
- 0,1-1 Gew.-% Magnesiumstearat
- 1-5 Gew.-% Feuchtigkeitsschutzbeschichtung.

8. Verfahren zur Herstellung der festen oralen pharmazeutischen Zusammensetzung gemäß Anspruch 7, umfassend die folgenden Schritte:
- Quellen von Polyvinylpyrrolidon in einer 70%igen Alkohollösung, die Ethanol und Wasser umfasst, wodurch eine Granulationslösung hergestellt wird,
- Mischen von mikrokristalliner Cellulose, vorgelatinierter Stärke, Croscarmellose-Natrium und Lurasidonhydrochlorid in einem Fließbetttrockner für 10 Minuten, um eine Pulvermischung herzustellen,
- Granulieren der Pulvermischung mit der Granulationslösung unter Verwendung eines Sprühgranulationsverfahrens,
- Trocknen der Granulate
- Sieben des Granulats und Mahlen desselben,
- Mischen der gemahlenen Granulate mit Mannitol für 15 Minuten in einem Behältermischer,
- Magnesiumstearat zu den mannitolisierten Granulaten hinzufügen und weitere 3 Minuten mischen,
- die fertige Mischung wiegen und zu Tabletten pressen,
- Beschichten dieser Tabletten mit einer Filmschicht.

## Revendications

1. - Composition pharmaceutique orale solide comprenant du chlorhydrate de lurasidone, au moins un désintégrant et deux liants qui sont l'amidon prégélatinisé et la polyvinylpyrrolidone, dans laquelle le rapport de l'amidon prégélatinisé au poids total du chlorhydrate de lurasidone est dans la plage de 1:2 à 1:3 et dans laquelle ledit désintégrant est la croscarmellose sodique et dans laquelle le rapport de la croscarmellose sodique au poids total du chlorhydrate de lurasidone est dans la plage de 1:2 et 1:3.

2. - Composition pharmaceutique orale solide selon la revendication 1, dans laquelle la composition comprend du chlorhydrate de lurasidone dans une quantité de 5 à 30 % et, de façon davantage préférée, de 10 à 20 % en poids.

3. - Composition pharmaceutique orale solide selon la revendication 1 ou 2, dans laquelle la composition est sous forme de comprimé enrobé, comprimé tricouche, comprimé bicouche, comprimé multicouche, comprimé à désintégration orale, mini-comprimé, pastille, granule de sucre, comprimé buccal, comprimé sublingual, comprimé effervescent, comprimé à libération immédiate, comprimé à libération modifiée, comprimé pelliculé, comprimé à désagrégation gastrique, pilule, capsule, granulé oral, poudre, système de perles enrobées, microsphère, comprimé dans comprimé, comprimé incrusté, dragée, sachet, film administrable par voie orale.

4. - Composition pharmaceutique orale solide selon la revendication 3, dans laquelle la composition se présente sous la forme d'un comprimé pelliculé.

5. - Composition pharmaceutique orale solide selon l'une quelconque des revendications précédentes, dans laquelle la composition comprend en outre au moins un excipient pharmaceutiquement acceptable choisi parmi les diluants, les désintégrants, les lubrifiants ou leurs mélanges.

6. - Composition pharmaceutique orale solide selon la revendication 1, dans laquelle le rapport de la croscarmellose sodique au poids total du liant est dans la plage de 1:0,5 à 1:10 et, de préférence, de 1:1 à 1:3 et, de façon davantage préférée, de 1:1 à 1:2.

7. - Composition pharmaceutique orale solide selon l'une quelconque des revendications précédentes, dans laquelle la composition comprend :
- 5 à 30% en poids de chlorhydrate de lurasidone
- 1 à 15% en poids d'amidon prégélatinisé
- 1 à 10% en poids de polyvinylpyrrolidone
- 5 à 60% en poids de cellulose microcristalline
- 1 à 15% en poids de croscarmellose sodique
- 5 à 60% en poids de mannitol
- 0,1 à 1% en poids de stéarate de magnésium
- 1 à 5% en poids d'un enrobage protecteur anti-humidité.

8. - Procédé de préparation de la composition pharmaceutique orale solide selon la revendication 7, comprenant les étapes suivantes consistant à :
- faire gonfler la polyvinylpyrrolidone dans une solution d'alcool à 70% comprenant de l'éthanol et de l'eau, permettant ainsi de préparer une solution de granulation ;
- mélanger la cellulose microcristalline, l'amidon prégélatinisé, la croscarmellose sodique et le chlorhydrate de lurasidone dans un séchoir à lit fluidisé pendant 10 minutes pour préparer un mélange pulvérulent ;
- granuler le mélange pulvérulent avec la solution de granulation à l'aide de la méthode de granulation par pulvérisation ;
- séchage des granulés ;
- tamiser les granulés et les broyer ;
- mélanger les granulés broyés avec du mannitol pendant 15 minutes dans un mélangeur à conteneur ;
- ajouter le stéarate de magnésium aux granulés mannitolisés et mélanger pendant 3 minutes supplémentaires ;
- peser le mélange final et le comprimer en comprimés ;
- enrober ces comprimés d'une couche de film.
